# EUROPEAN PATENT SPECIFICATION

(11) **EP 3 052 056 B1**
(45) Date of publication and mention of the grant of the patent: **05.07.2017**
(21) Application number: 14777132.3
(22) Date of filing: 29.09.2014
(51) Int. Cl.: A61F 2/46, A61F 2/34

(54) **AN INSTRUMENT FOR POSITIONING A CUP COMPONENT OF AN ORTHOPAEDIC JOINT PROSTHESIS**
INSTRUMENT ZUR POSITIONIERUNG EINER PFANNENKOMPONENTE EINER ORTHOPÄDISCHEN GELENKPROTHESE
INSTRUMENT POUR LE POSITIONNEMENT D'UN ÉLÉMENT DE COQUE D'UNE PROTHÈSE ARTICULAIRE ORTHOPÉDIQUE

(30) Priority: 30.09.2013 GB 201317285
(43) Date of publication of application: 10.08.2016
(73) Proprietor: DePuy Ireland Unlimited Company, County Cork (IE)
(72) Inventor: BAILEY, Andrew, Leeds LS11 8DT (GB)
(74) Representative: Alton, Andrew
(86) International application number: PCT/GB2014/052928
(87) International publication number: WO 2015/044680

(56) References cited:
- DE-A1- 10 250 390
- GB-A- 2 299 758
- US-A- 5 108 448
- US-A- 5 683 399

## Description

This invention relates to an instrument for positioning a cup component of an orthopaedic joint prosthesis.

Certain orthopaedic joint prostheses include a hollow cup with an inner surface which defines a generally hemispherical hollow region, and another component which has a spherical part which can be received in the hollow region for articulation relative to the cup component. Such joint prostheses can include hip joint prostheses and shoulder joint prostheses. The exterior of the cup will contact the prepared surface of the patient's bone in which the component is to be implanted. The interior of the cup will present a smooth bearing surface to the spherical part of the other component of the joint prosthesis. The bearing surface can be provided by a single piece cup component. Alternatively, the cup component can comprise a shell part which contacts the prepared surface of the patient's bone, and a bearing part which provides the bearing surface, and which fits into the shell part. The bearing part can be made from a material which is different from the material of the shell part: for example the bearing part can be made from a polymeric material (such as polyethylene) or a ceramic material and the shell part (and the spherical part of the other component) can be made from a metal (such as a cobalt-chromium based alloy, or a stainless steel, or a titanium based alloy).

It is important that the components of an orthopaedic joint prosthesis are positioned accurately in a patient's bone. Both location and alignment are important. Accurate positioning of a component requires that the component be engaged by an appropriate instrument, allowing considerable force to be applied to the component if and as necessary. However, it can be important not to contact the external surface or the internal surface or both of the component with the instrument, especially the internal surface when it has been provided with a smooth polished bearing surface. Scratching or otherwise damaging that surface can impair the bearing properties of the prosthesis.

US-5171243 discloses an acetabular cup for use in a hip joint prosthesis. The cup comprises a shell which has a circumferential groove cut into its inner surface. The groove can received a flange at the free end of an insertion tool so that the cup is retained on the instrument, allowing the shell to be manipulated using the instrument. The grooved shell part receives a bearing part which has a smooth inner surface against which a bearing surface of another component of the joint prosthesis can articulate. The shell part can have fastening holes extending through its wall through which bone screws can extend to fasten the shell part to the surface of a bone.

WO-A-2008/099242 discloses an instrument for gripping a cup component of an orthopaedic joint prosthesis which includes a plurality of jaw members which extend radially from a central drive shaft. The jaw members can be made to slide radially inwardly so that they engage the outside wall of a cup component. Each of the jaw members has a pin at one end which is received in a spiral track on a drive plate. The jaw members are made to slide radially by rotating the drive plate.

GB-2,999,758 describes a cup handling instrument in which respective pins within curved slots are driven against curved surfaces defining the curved slots to cause gripping formations at the ends of jaws to be driven outwardly to engage with the periphery of a deformable acetabular cup. The parts of the jaws which engage with the outer and inner surface of the cup define a slot which receives the peripheral edge of the deformable cup.

DE-10250390 describes an expander element which can be located within a spreader plug and to drive separate segments of the plug outwardly to engage matching recesses provided on the inner surface of an acetabular component.

US 5,683,399 describes an acetabular cup insertion tool having a collet at a free end and flat surfaces against which an inner spreader component is driven to drive outwardly directed features to engage within a recess within an interior of an acetabular cup.

US-5,108,448 describes an instrument for inserting an acetabular cup having a plurality of levers which are pivoted on pins and have two hooks which extend radially by longitudinal movement of a control rod which causes triangular projections to ride over each other and drive the hooks outward.

A first aspect of the invention provides an instrument for positioning a cup component of an orthopaedic joint prosthesis, comprising: an actuator; and a plurality of jaw members arranged around a longitudinal axis of the instrument, each jaw member having: a mounting end, a gripping end with a transversely extending member directed inwardly which can be received in a recess in an outwardly facing side wall of a cup component, wherein the gripping end of each of the jaw members includes a flat underside surface arranged to engage an upper surface of a rim of the cup component and can be displaced between a retracted position and a deployed position in which the gripping end is displaced from the retracted position in the direction in which the transversely extending member is directed, and a surface between the mounting end and the gripping end which is inclined to the longitudinal axis of the instrument and curved in an outward direction away from the longitudinal axis and in a peripheral direction around the longitudinal axis, wherein the actuator and the plurality of jaw members are capable of relative movement along the longitudinal axis of the instrument with the actuator in contact with the inclined surfaces of the jaw members so that said relative movement causes the jaw members to move between their retracted position and deployed position.

The transversely extending members at the gripping ends of the jaw members are directed inwardly, so that they can then be received in one or more recesses which are provided in the outwardly facing side wall of the cup component. The jaw members have a surface towards the gripping ends of the jaw members which faces generally along the axis of the instrument, towards a cup component which is engaged by the jaw members. The surface can act against a rim of the cup component in use so that an impaction force that is applied to the instrument is transmitted to the cup component.

The instrument of the invention has the advantage that the number of parts from which it is made is small. Furthermore, assembly of the instrument from its parts can be simple. These advantages mean that the instrument can be manufactured simply and at low cost and is easy to clean.

The surface between the mounting end and gripping end may transition as a smooth and/or continuous curve extending from the mounting end to the gripping end.

The flat underside surfaces may all be perpendicular to the longitudinal axis of the instrument. Each flat underside surface may be positioned between a respective transversely extending member and the longitudinal axis of the instrument. The flat underside surfaces may extend in a peripheral direction sufficiently to form a complete annular underside surface when the jaw members are in the deployed position.

Optionally, the actuator can be a hollow sleeve and the jaw members can extend within the actuator. The inclined surfaces of the jaw members can then be outwardly facing surfaces of the jaw members which contact an inwardly facing surface of the actuator.

The actuator can be in the form of a hollow cylinder. The cylinder can have a circumferentially continuous wall, although the invention can be implemented using an actuator in the form of a cylinder which has slots formed in its wall. The cylinder might be short so that it might be viewed as a collar. The jaw members can be arranged within the bore of an actuator which is in the form of a hollow cylinder.

Optionally, the jaw members are biassed resiliently towards their retracted positions. In an instrument which has this feature, the actuator can be used to apply a force to the jaw member against the bias, to cause them to move towards their deployed positions. The jaw members can move towards their retracted positions by moving the actuator and allowing the jaw members to move as a result of the biassing force. This can be a preferred arrangement because it means that the cup component is engaged positively by the instrument.

The jaw members can be biassed resiliently towards their deployed positions. In an instrument which has this feature, the actuator can be used to apply a force to the jaw member against the bias, to cause them to move towards their retracted positions. The jaw members can move towards their deployed positions by moving the actuator and allowing the jaw members to move as a result of the biassing force.

Optionally, the jaw members can be provided by a component which flares outwardly towards the gripping ends of the jaw members. The component can have generally conical side walls. The inclined surfaces of the jaw members can be curved when the jaw members are viewed tangentially so that, for example, the component can be approximately bell-shaped when viewed from the side. The jaw members can be formed in the component by slots in the wall of the component which are open at the gripping end of the jaw members. The slots can extend from the gripping ends of the jaw members towards but not as far as the top of the component. The flared side walls of the component can be acted on by a hollow actuator which acts on the outwardly facing surfaces of the side walls.

The slots between adjacent jaw members can be relatively narrow. For example, when the cup component is circular so that the jaw members form a circle, it can be preferred that the ratio of the angle subtended at the axis of the instrument by each jaw member to the angle subtended by a slot between two adjacent jaw members is at least about 3, more preferably at least about 4.

The transversely extending member on each of the jaw members can have the same width as the jaw member itself so that the transversely extending member on each of the jaw members subtends the same angle at the axis of the instrument as the jaw member on which it is located.

The transversely extending member on each of the jaw members can be narrower than the jaw member so that the angle subtended by the transversely extending member on each of the jaw members at the axis of the instrument is smaller than the angle subtended by the jaw member on which it is located. For example, when the cup component is circular so that the jaw members form a circle, the ratio of the angle subtended at the axis of the instrument by the transversely extending member on each jaw member to the angle subtended by the jaw member can be less than about 0.5, or less than 0.3, for example less than 0.15.

The component can comprise a tube whose wall is flared outwardly and has slots formed in it at one end.

Optionally, the instrument includes a handle. Optionally, the jaw members are attached to the handle, and the actuator can be moved relative to the handle and the jaw members.

The instrument can have an impaction surface through which an impaction force can be applied to the instrument and to a cup component which is attached to the instrument. The instrument can include a central shaft which is in communication with the impaction surface to transfer the impaction force to the cup component. This can be used to force the cup component into tight fitting engagement with a prepared bone cavity.

Optionally, the instrument includes an actuator mechanism for causing the actuator to move relative to the handle and to the jaw members. The actuator mechanism can include a driver which is mounted so that rotation of the driver causes displacement of the actuator along the longitudinal axis of the instrument relative to the handle. The driver can have a sloped or tilted end face and the actuator can have a sloped or tilted end face against which the driver end face abuts. The degree of tilt or slope of the end faces of the driver and actuator relative to the longitudinal axis of the instrument can be the same.

The actuator mechanism can have other forms. For example, the actuator mechanism can include a camming arrangement, a threaded arrangement, a pin travelling in a helical slot, a wedge or a simple push action.

The instrument can have two jaw members. Preferably, the instrument has more than two jaw members. A preferred embodiment has three or four jaw members. The instrument can have up to ten jaw members.

Preferably the jaw members are arranged so that they are spaced apart uniformly around a cup component with which they are to be used. For example, when the instrument has three jaw members and the cup component is circular, the angle subtended between adjacent jaw members at the longitudinal axis of the instrument can be 120°. When the instrument has four jaw members and the cup component is circular, the angle subtended between adjacent jaw members at the longitudinal axis of the instrument can be 90°.

Component parts of the instrument can be made from polymeric materials or metallic materials or from both polymeric and metallic materials. Suitable polymeric materials include polyolefins, polyesters, engineering polymers such as polyether-ether-ketones, and acetals. Suitable metals include certain stainless steels, and titanium and its alloys. The use of polymeric materials for at least some of the component parts of the instrument has the advantage of lower cost and ease of manufacture, for example by moulding.

For many applications, the cup component will be rotationally symmetrical about a polar axis. It will often be preferred that the cup component has the shape of a portion of a sphere. As is known, the rim of a cup component to be fitted in a patient's acetabulum in a hip replacement procedure will frequently subtend an angle of at least 140° at the centre of the sphere, for example at least 150° or at least 160° or at least 170°. The rim of an acetabular cup component will frequently be circular, although sometimes the rim can have an extension on one side for example to reduce the risk of dislocation. When the rim of the cup component is circular, the rim can be planar around at least 50% of the periphery of the cup component. The plane defined by the rim can be perpendicular to the polar axis of the component.

The glenoid component of a shoulder prosthesis will generally be more shallow than the cup component of a hip prosthesis and its rim might not be circular.

The bone facing surface of the cup component can be adapted for contact with bone tissue. For example, it can be adapted to bond to the bone tissue as a result of direction interaction (for example bone ingrowth) of bone into the surface of the component, or it can be adapted to bond to the tissue by means of a bone cement material.

The cup component can have a plurality of discrete recesses arranged around its periphery in which the transversely extending members on the jaw members can be received. When the cup component has discrete recesses arranged around its periphery, the number of recesses should not be less than the number of jaw members. Frequently, the number of recesses and the spacing between the recesses should correspond to the number of transversely extending members and the spacing between the fingers.

The cup component can have a recess which is longer than each of the transversely extending members which are received in it, for example in the form of a groove which extends around at least part of the cup component. The groove can extend continuously around the entire periphery of the cup component.

The invention also provides an assembly for use in a surgical procedure to replace an orthopaedic joint, which comprises: the instrument of the first aspect and a cup component of an orthopaedic joint prosthesis, in which each of the transversely extending members on the jaw members of the instrument are received in a respective recess in the outwardly facing side wall of the cup component, and wherein the flat underside surfaces engage the upper surface of the rim of the wall of the cup component.

Embodiments of the invention are described below in detail, by way of example only, and with reference to the accompanying drawings, in which:
Figure 1 is an isometric view of a cup component and an instrument for positioning the cup component according to the invention;
Figure 2 is a cross sectional side view of the cup component and the instrument shown in Figure 1;
Figure 3 is an isometric view of an actuator mechanism part of the instrument shown in Figures 1 and 2;
Figure 4 is an isometric view of an actuator part of the instrument shown in Figures 1 and 2;
Figure 5 is an isometric view of a cup holding part of the instrument shown in Figures 1 and 2;
Figure 6 is an isometric view of the cup component shown in Figures 1 and 2; and
Figure 7 is an isometric view of a further embodiment of a cup holder part of the instrument and a cup component.

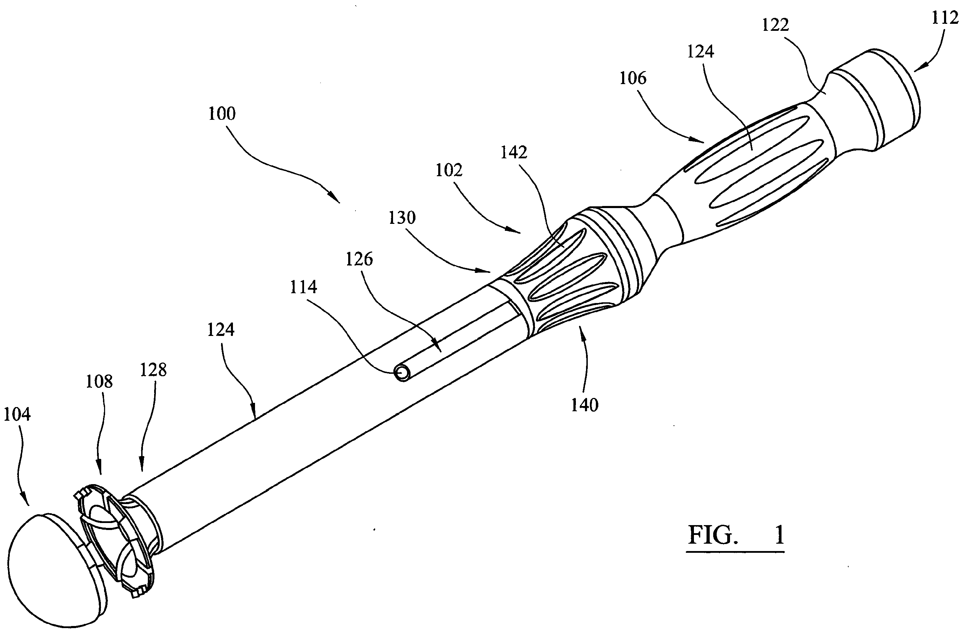

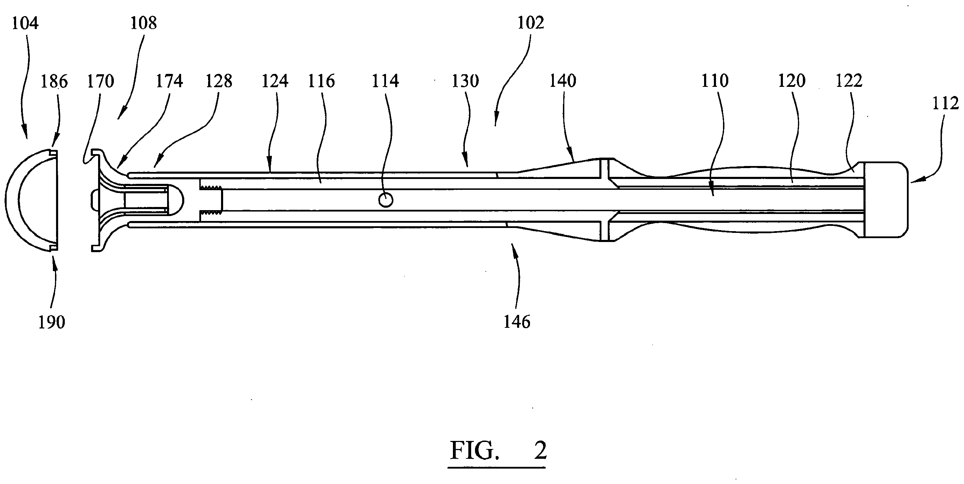

With reference to Figure 1 there is shown an isometric view of an instrument 102 for positioning a cup component 104 of an orthopaedic joint prosthesis. The instrument 102 and cup 104 can be combined to form an assembly 100. Figure 2 shows a transverse cross section along a longitudinal axis of the instrument 102 and cup component 104.

The instrument 102 includes a handle 106 at a proximal end, and a cup holding part 108 at a distal end. As best illustrated in Figure 2, the instrument 102 has a central shaft 110 extending along its longitudinal axis and terminating at the proximal end in an impaction head 112. A first peg 114 and a second peg (not shown) extend transversely along its longitudinal axis from the central shaft. A sleeve 116 of a metal or engineering polymer surrounds shaft 110. A distal end of sleeve 116 defines a blind threaded bore by which the cup holding component 108 is attached to the instrument.

A further sleeve 120 of a metal or an engineering polymer surrounds a more proximal part of shaft 110 and handle 106 is formed by a grip 122 surrounding the shaft. The grip 122 may be formed of any suitable material, such as a silicone rubber or an engineering polymer. The grip 122 can include a plurality of longitudinal recesses, e.g. recess 124, to provide an enhanced grip for the handle part 106 of the instrument.

An actuator is provided between the handle 106 and cup holding component 108. The actuator includes a cylindrical tube 124 having a longitudinally extending closed slot, e.g. slot 126, in each side and for accepting each of the pair of pins, 114, which act to prevent rotation of the tube 124. A distal end 128 of push shaft 124 encircles and surrounds the cup holding component 108. A proximal end 130 of the push shaft 124 has end surfaces which are tilted relative to the longitudinal axis of the instrument in that they do not subtend an angle of 90° relative to the longitudinal axis of the instrument.

A rotatable collar 140 provides an actuator mechanism and is journalled about sleeve 116 between the push shaft 124 and handle 106. Collar 140 includes a number of longitudinal recesses, e.g. 142, in its outer surface to provide grip formations. The collar 140 is generally tubular and tapers from a greater outer diameter at a proximal end to a lesser outer diameter at a distal end 146. Distal end 146 provides an end surface 148 defining a plane which is tilted relative to the longitudinal axis of the instrument, in that it subtends an angle different to 90° and is not perpendicular to the longitudinal axis. The end face 148 of the collar subtends the same angle as the end face of the push shaft such that they are parallel, as illustrated in Figure 1. The angle subtended by the end face of the collar / end face of the push shaft depends on the force needed to hold the cup.

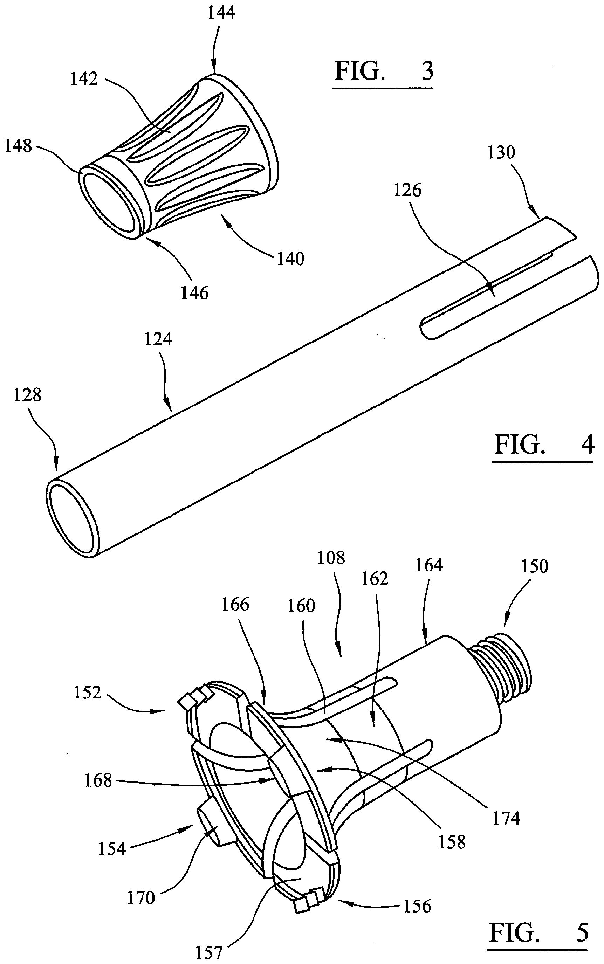

The cup holding component 108 is shown in isolation in Figure 5 and generally has the form of a flared, rotationally symmetric cylinder. A threaded boss 150 extends from a proximal end. The cup holding component 108 includes four jaw members 152, 154, 156, 158. Each jaw member 152, 154, 156, 158 is defined by a pair of slits, e.g. slit 160 extending through the wall of the component. Each slit can be a few mm wide, for example about 1mm wide. Each jaw member includes a mounting end, e.g. 162 by which the jaw member includes a mounting end, e.g. 162 by which the jaw member is mounted to the main body 164 of the component. Each jaw member also includes a gripping end 166 which bears a member, 168 arranged to engage a corresponding recess in the cup component 104. Each member 168 extends at least partially in a direction transverse to the longitudinal axis of the instrument and presents an inwardly facing slightly sloped surface, e.g. 170. Each jaw member includes a surface, e.g. 174 extending between the gripping end and the mounting end and which is inclined relative to the longitudinal axis of the instrument. As best illustrated in Figures 1 and 2, an inner surface of the distal end 128 of the actuator 124 engages the inclined surface 174 and can be urged there over when the actuator 124 is translated along the longitudinal axis of the instrument as described in greater detail below.

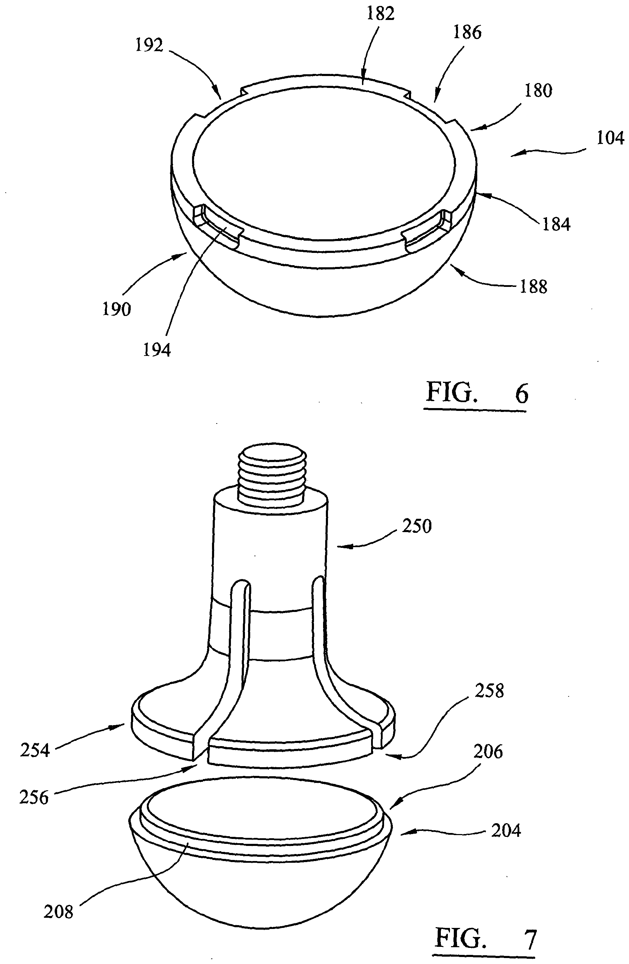

Figure 6 shows a perspective view of the cup component 104. As illustrated in Figure 6, the cup 104 has a generally hemispherical shape and includes a rim portion 180 with an upper surface 182 and a peripheral side surface 184. Four recesses 186, 188, 190, 192 and a peripheral side surface 184. Four recesses 186, 188, 190, 192 are formed in an outer surface of the rim portion at equi-angularly spaced positions. Each recess, 186, 188, 190, 192 is positioned, sized and spaced to accept a respective one of the transverse extending members 152, 154, 156, 158. An inner surface e.g. 194 tilts inwardly slightly to form a recess into which the transversely extending member can be received and to abut against the inner wall 170 of each transversely extending member.

Operation of the instrument will now be described. Figure 1 shows the jaw members in a retracted position in which the transversely extending members 152, 154, 156, 158 can be introduced into a respective recess 186, 188, 190, 192 in the rim of the cup component 104. The cup holding member 108 is made from a plastic material and is resiliently biased so that the jaw members are naturally held in the retracted position. In order to securely lock the cup component to the instrument 102, a user can operate the actuator mechanism by rotating collar 140 about the longitudinal axis of the instrument. The tilted end surface of the collar 140 acts against the tilted end surfaces of the proximal portion 130 of the push shaft. The pair of pegs engaged in the pair of slots in the push shaft constrain the push shaft to prevent its rotation such that the push shaft is caused to translate along the longitudinal axis of the instrument and toward the cup holding component 108. The distal end 128 of the push shaft 124 drives against the inclined surface 174 of each jaw member and causes the gripping ends of each jaw member to be displaced in a generally transverse direction into a deployed position in which the transversely extending members are urged into the recesses in the cup component and into abutment with the inner surfaces of each recess, e.g. 194. As the inner surface of each transversely extending member and recess are tilted slightly relative to the longitudinal axis, this provides a recess and tooth arrangement which prevents the cup from being released from the instrument in a longitudinal direction.

The instrument can then be used to manipulate the cup component into position and an impactor can be used to impart an impaction force on the cup instrument by impacting impaction head 112 of the instrument. As the impaction head is attached to the central shaft 110 of the instrument, this helps to transfer the impaction force along the instrument and on to the upper surface of the rim of the cup 182 via the flat underside surfaces, e.g. 157, of the gripping end of each jaw member.

In order to remove the instrument from the cup component 104, the actuator mechanism is rotated in the opposite sense which allows the actuator shaft 124 to travel along the longitudinal axis by the action of the inclined surfaces of each jaw, being resiliently biased toward the retracted position, acting on the distal end 128 of the actuator shaft 124. Hence, the jaw members can retain the original retractor position in which the transversely extending members are no longer received within the recesses in the cup and so the instrument can be removed from the cup component.

Figure 7 shows a further embodiment of the cup holding component 250 and cup component 204. The cup holding component 250 is similar in construction to that shown in Figure 5 other than the form of the transversely extending members. In the second embodiment of the cup holding member 250, each transversely extending member, 252, 254, 256, 258 extends along the entire peripheral length of the gripping end of each jaw member. The cup component 204 includes a single recess 206 in the rim of the cup component and extending around the entire periphery of the cup component 204.

Hence, in the retracted position, as illustrated in Figure 7, the transversely extending members, which have a generally arcuate shape, can all be introduced into the same single recess 206 and the relative angular position of the cup and cup holding component 250 does not need to be carefully controlled. The actuator mechanism, when operated, causes the transversely extending members to move toward the deployed position in which an inner surface of the transversely extending members abuts an inner surface 208 of recess 206. Inner surface 208 is inclined slightly relative to the longitudinal axis as is an inner surface of the transversely extending members so as to prevent the cup component becoming detached by movement in a longitudinal direction.

Operation of the instrument and its actuation are largely described above. All that is varied are the details of the mechanism by which the cup component can be releasably and securely attached to the cup holding component of the instrument.

## Claims

1. An instrument (102) for positioning a cup component (104) of an orthopaedic joint prosthesis, comprising:
an actuator (124); and
a plurality of jaw members (152, 154, 156, 158) arranged around a longitudinal axis of the instrument, each jaw member having:
a mounting end (162),
a gripping end (166) with a transversely extending member (168) directed inwardly which can be received in a recess (186, 188, 190, 192) in an outwardly facing side wall (184) of a cup component (104), wherein the gripping end of each of the jaw members includes a flat underside surface (157) arranged to engage an upper surface (182) of a rim of the cup component and can be displaced between a retracted position and a deployed position in which the gripping end is displaced from the retracted position in the direction in which the transversely extending member is directed, and
a surface (174) between the mounting end (162) and the gripping end (166) which is inclined to the longitudinal axis of the instrument and curved in an outward direction away from the longitudinal axis and in a peripheral direction around the longitudinal axis, wherein the actuator and the plurality of jaw members are capable of relative movement along the longitudinal axis of the instrument with the actuator (124) in contact with the inclined surfaces of the jaw members so that said relative movement causes the jaw members to move between their retracted position and deployed position.

2. The instrument (102) as claimed in claim 1, wherein the surface (174) extends as a continuous curve between the mounting end (162) and the gripping end (166).

3. The instrument (102) as claimed in claim 1, wherein the flat underside surfaces (157) are all perpendicular to the longitudinal axis of the instrument.

4. The instrument (102) of claim 1 or 3, wherein each flat underside surface (157) is positioned between a respective transversely extending member (168) and the longitudinal axis of the instrument.

5. The instrument (102) of any of claims 1 to 4, wherein the flat underside surfaces (157) extend in a peripheral direction sufficiently to form a complete annular underside surface when the jaw members are in the deployed position.

6. The instrument (102) as claimed in any of claims 1 to 5, in which the actuator (124) is a hollow sleeve and the jaw members (152, 154, 156, 158) extend within the actuator, the inclined surfaces (174) of the jaw members being outwardly facing surfaces of the jaw members which contact an inwardly facing surface of the actuator.

7. The instrument (102) as claimed in any of claims 1 to 6, in which the jaw members (152, 154, 156, 158) are biassed resiliently towards their retracted positions.

8. The instrument (102) as claimed in any of claims 1 to 7, in which each transversely extending member (252, 254, 256, 258) extends along substantially the entire peripheral length of each respective gripping end.

9. The instrument (102) as claimed in any of claims 1 to 8, in which the jaw members (152, 154, 156, 158) are provided by a component (108) having a wall which flares outwardly towards the gripping ends of the jaw members, and having slits (160) in the wall, each slit being open at the gripping ends (166) of the jaw members.

10. The instrument (102) as claimed in any of claims 1 to 9, and including a handle (106), and in which the jaw members (152, 154, 156, 158) are attached to the handle, and in which the actuator (124) can be moved relative to the handle and the jaw members, and the instrument further including an actuator mechanism (140) operable to move the actuator (124) relative to the handle (106) and to the jaw members, and in which the actuator mechanism (140) includes a rotatable driver (140) coupled to the actuator (124) and wherein rotation of the driver (140) causes displacement of the actuator (124) along the longitudinal axis of the instrument relative to the handle.

11. The instrument (102) as claimed in claim 10, wherein the rotatable driver (140) includes a first end face (148) which is tilted relative to the longitudinal axis and the actuator (124) includes a second end face which is parallel to the first end face (148) and wherein the first and second end face abut to form the coupling between the rotatable driver (140) and the actuator (124).

12. The instrument (102) as claimed in claim 11, wherein the actuator (124) includes an anti-rotation mechanism (114) which prevents rotation of the actuator relative to the instrument about the longitudinal axis but allows translation along the longitudinal axis.

13. The instrument (102) as claimed in claim 1, wherein the instrument has an impaction surface (112) through which an impaction force can be applied to the instrument and a central shaft (110) which is in communication with the impaction surface to transfer the impaction force to the cup component.

14. The instrument (102) as claimed in any of claims 1 to 14, wherein the plurality of jaw members (152, 154, 156, 158) are parts of a cup holding member (108) made from a plastic material.

15. An assembly (100) for use in a surgical procedure to replace an orthopaedic joint, which comprises:
the instrument (102) as claimed in any of claims 1 to 14, and
a cup component (104) of an orthopaedic joint prosthesis, in which each of the transversely extending members on the jaw members of the instrument are received in a respective recess (186, 188, 190, 192) in the outwardly facing side wall (184) of the cup component, and wherein the flat underside surfaces engage the upper surface (182) of the rim of the wall of the cup component.

## Patentansprüche

1. Instrument (102) zur Positionierung einer Pfannenkomponente (104) einer orthopädischen Gelenkprothese, umfassend:
einen Betätiger (124); und
eine Vielzahl von Backenelementen (152, 154, 156, 158), welche um eine Längsachse des Instruments angeordnet sind, wobei jedes Backenelement enthält:
ein Befestigungsende (162),
ein Greifende (166) mit einem einwärts gerichteten quer verlaufenden Element (168), welches in einer Vertiefung (186, 188, 190, 192) in einer nach außen gewandten Seitenwand (184) einer Pfannenkomponente (104) aufgenommen werden kann, wobei das Greifende jedes der Backenelemente eine flache Unterseitenfläche (157) enthält, welche so angeordnet ist, dass sie mit einer oberen Fläche (182) eines Rands der Pfannenkomponente in Eingriff tritt, und zwischen einer eingezogenen Stellung und einer ausgezogenen Stellung verschoben werden kann, in welcher das Greifende aus der eingezogenen Stellung in die Richtung verschoben ist, in welche das quer verlaufende Element gerichtet ist, und
eine Fläche (174) zwischen dem Befestigungsende (162) und dem Greifende (166), welche zu der Längsachse des Instruments geneigt und in einer Auswärtsrichtung von der Längsachse weg und in einer Umfangsrichtung um die Längsachse gekrümmt ist, wobei der Betätiger und die Vielzahl von Backenelementen zu einer Relativbewegung entlang der Längsachse des Instruments in der Lage sind, wobei der Betätiger (124) mit den geneigten Flächen der Backenelemente in Kontakt steht, sodass diese Relativbewegung die Backenelemente veranlasst, sich zwischen ihrer eingezogenen Stellung und ausgezogenen Stellung zu bewegen.

2. Instrument (102) nach Anspruch 1, wobei die Fläche (174) als eine durchgehende Kurve zwischen dem Befestigungsende (162) und dem Greifende (166) verläuft.

3. Instrument (102) nach Anspruch 1, wobei die flachen Unterseitenflächen (157) alle senkrecht zu der Längsachse des Instruments sind.

4. Instrument (102) nach Anspruch 1 oder 3, wobei jede flache Unterseitenfläche (157) zwischen einem entsprechenden quer verlaufenden Element (168) und der Längsachse des Instruments angeordnet ist.

5. Instrument (102) nach einem der Ansprüche 1 bis 4, wobei die flachen Unterseitenflächen (157) ausreichend in einer Umfangsrichtung verlaufen, um eine vollständige ringförmige Unterseitenfläche zu bilden, wenn die Backenelemente in der ausgezogenen Stellung sind.

6. Instrument (102) nach einem der Ansprüche 1 bis 5, bei dem der Betätiger (124) eine hohle Muffe ist und die Backenelemente (152, 154, 156, 158) in dem Betätiger verlaufen, wobei die geneigten Flächen (174) der Backenelemente nach außen gewandte Flächen der Backenelemente sind, welche eine nach innen gewandte Fläche des Betätigers berühren.

7. Instrument (102) nach einem der Ansprüche 1 bis 6, bei dem die Backenelemente (152, 154, 156, 158) nachgiebig in ihre eingezogenen Stellungen vorgespannt sind.

8. Instrument (102) nach einem der Ansprüche 1 bis 7, bei dem jedes quer verlaufende Element (252, 254, 256, 258) entlang im Wesentlichen der gesamten Umfangslänge jedes entsprechenden Greifendes verläuft.

9. Instrument (102) nach einem der Ansprüche 1 bi 8, bei dem die Backenelemente (152, 154, 156, 158) durch eine Komponente (108) bereitgestellt sind, welche eine Wand hat, die nach außen zu den Greifenden der Backenelemente hin aufweitet, und welche Schlitze (160) in der Wand hat, wobei jeder Schlitz an den Greifenden (166) der Backenelemente offen ist.

10. Instrument (102) nach einem der Ansprüche 1 bis 9 und einen Griff (106) umfassend, und bei dem die Backenelemente (152, 154, 156, 158) an dem Griff angebracht sind, und bei dem der Betätiger (124) relativ zu dem Griff und den Backenelementen bewegt werden kann, und wobei das Instrument ferner einen Betätigermechanismus (140) enthält, welcher zum Bewegen des Betätigers (124) relativ zu dem Griff (106) und zu den Backenelementen betätigbar ist, und bei dem der Betätigermechanismus (140) eine drehbare Treibvorrichtung (140) enthält, welche an den Betätiger (124) gekoppelt ist, und wobei eine Drehung der Treibvorrichtung (140) das Verschieben des Betätigers (124) entlang der Längsachse des Instruments relativ zu dem Griff bewirkt.

11. Instrument (102) nach Anspruch 10, wobei die drehbare Treibvorrichtung (140) eine erste Endfläche (148) enthält, welche relativ zu der Längsachse geneigt ist, und der Betätiger (124) eine zweite Endfläche enthält, welche parallel zu der ersten Endfläche (148) ist, und wobei die erste und die zweite Endfläche anstoßen, um die Kopplung zwischen der drehbaren Treibvorrichtung (140) und dem Betätiger (124) zu bilden.

12. Instrument (102) nach Anspruch 11, wobei der Betätiger (124) einen Drehsicherungsmechanismus (114) enthält, welcher die Drehung die Betätigers relativ zu dem Instrument um die Längsachse verhindert, aber die Verschiebung entlang der Längsachse erlaubt.

13. Instrument (102) nach Anspruch 1, wobei das Instrument eine Stoßfläche (112), durch die eine Stoßkraft auf das Instrument ausgeübt werden kann, und einen zentralen Schaft (110) hat, welcher zum Übertragen der Stoßkraft auf die Pfannenkomponente mit der Stoßfläche in Verbindung steht.

14. Instrument (102) nach einem der Ansprüche 1 bis 14, wobei die mehreren Backenelemente (152, 154, 156, 158) Teile eines aus einem Kunststoffmaterial bestehenden Pfannenhalteelements (108) sind.

15. Baugruppe (100) zur Verwendung bei einem chirurgischen Eingriff zum Ersetzen eines orthopädischen Gelenks, welche umfasst:
das Instrument (102) nach einem der Ansprüche 1 bis 14, und
eine Pfannenkomponente (104) einer orthopädischen Gelenkprothese, wobei jedes der quer verlaufenden Elemente an den Backenelementen des Instruments in einer entsprechenden Vertiefung (186, 188, 190, 192) in der nach außen gewandten Seitenwand (184) der Pfannenkomponente aufgenommen ist, und wobei die flachen Unterseitenflächen mit der oberen Fläche (182) des Rands der Wand des Pfannenkomponente in Eingriff stehen.

## Revendications

1. Instrument (102) pour positionner un composant de cupule (104) d'une prothèse articulaire orthopédique, comprenant :
un actionneur (124) ; et
une pluralité d'éléments de mâchoire (152, 154, 156, 158) qui sont agencés autour d'un axe longitudinal de l'instrument, chaque élément de mâchoire comportant :
une extrémité de montage (162) ;
une extrémité de préhension (166) munie d'un élément s'étendant transversalement (168) dirigé vers l'intérieur qui peut être reçu dans un évidement (186, 188, 190, 192) dans une paroi latérale orientée vers l'extérieur (184) d'un composant de cupule (104), dans lequel l'extrémité de préhension de chacun des éléments de mâchoire inclut une surface inférieure plane (157) qui est agencée de manière à engager une surface supérieure (182) d'un bord du composant de cupule et peut être déplacée entre une position rétractée et une position déployée dans laquelle l'extrémité de préhension est déplacée par rapport à la position rétractée dans la direction dans laquelle l'élément s'étendant transversalement est dirigé ; et
une surface (174) entre l'extrémité de montage (162) et l'extrémité de préhension (166), laquelle surface est inclinée par rapport à l'axe longitudinal de l'instrument et est incurvée dans une direction vers l'extérieur à distance de l'axe longitudinal et dans une direction périphérique autour de l'axe longitudinal, dans lequel l'actionneur et les éléments de la pluralité d'éléments de mâchoire sont capables d'un déplacement relatif suivant l'axe longitudinal de l'instrument avec l'actionneur (124) qui est en contact avec les surfaces inclinées des éléments de mâchoire de sorte que ledit déplacement relatif ait pour effet que les éléments de mâchoire se déplacent entre leur position rétractée et leur position déployée.

2. Instrument (102) selon la revendication 1, dans lequel la surface (174) s'étend en tant que courbe continue entre l'extrémité de montage (162) et l'extrémité de préhension (166).

3. Instrument (102) selon la revendication 1, dans lequel les surfaces inférieures planes (157) sont toutes perpendiculaires à l'axe longitudinal de l'instrument.

4. Instrument (102) selon la revendication 1 ou 3, dans lequel chaque surface inférieure plane (157) est positionnée entre un élément s'étendant transversalement respectif (168) et l'axe longitudinal de l'instrument.

5. Instrument (102) selon l'une quelconque des revendications 1 à 4, dans lequel les surfaces inférieures planes (157) s'étendent dans une direction périphérique de manière suffisante pour former une surface inférieure annulaire complète lorsque les éléments de mâchoire sont dans la position déployée.

6. Instrument (102) selon l'une quelconque des revendications 1 à 5, dans lequel l'actionneur (124) est une gaine creuse et les éléments de mâchoire (152, 154, 156, 158) s'étendent à l'intérieur de l'actionneur, les surfaces inclinées (174) des éléments de mâchoire étant des surfaces orientées vers l'extérieur des éléments de mâchoire qui entrent en contact avec une surface orientée vers l'intérieur de l'actionneur.

7. Instrument (102) selon l'une quelconque des revendications 1 à 6, dans lequel les éléments de mâchoire (152, 154, 156, 158) sont poussés de manière élastique en direction de leurs positions rétractées.

8. Instrument (102) selon l'une quelconque des revendications 1 à 7, dans lequel chaque élément s'étendant transversalement (252, 254, 256, 258) s'étend suivant sensiblement la totalité de la longueur périphérique de chaque extrémité de préhension respective.

9. Instrument (102) selon l'une quelconque des revendications 1 à 8, dans lequel les éléments de mâchoire (152, 154, 156, 158) sont constitués par un composant (108) ayant une paroi qui s'évase vers l'extérieur en direction des extrémités de préhension des éléments de mâchoire, et qui comporte des fentes (160) qui sont ménagées dans la paroi, chaque fente étant ouverte au niveau des extrémités de préhension (166) des éléments de mâchoire.

10. Instrument (102) selon l'une quelconque des revendications 1 à 9, et incluant un manche (106), et dans lequel les éléments de mâchoire (152, 154, 156, 158) sont fixés au manche, et dans lequel l'actionneur (124) peut être déplacé par rapport au manche et par rapport aux éléments de mâchoire, et l'instrument incluant en outre un mécanisme d'actionneur (140) qui peut opérer pour déplacer l'actionneur (124) par rapport au manche (106) et par rapport aux éléments de mâchoire, et dans lequel le mécanisme d'actionneur (140) inclut un dispositif d'entraînement rotatif (140) qui est couplé à l'actionneur (124) et dans lequel la rotation du dispositif d'entraînement (140) provoque un déplacement de l'actionneur (124) suivant l'axe longitudinal de l'instrument par rapport au manche.

11. Instrument (102) selon la revendication 10, dans lequel le dispositif d'entraînement rotatif (140) inclut une première face d'extrémité (148) qui est inclinée par rapport à l'axe longitudinal et l'actionneur (124) inclut une seconde face d'extrémité qui est parallèle à la première face d'extrémité (148) et dans lequel les première et seconde faces d'extrémité viennent en butée de manière à former le couplage entre le dispositif d'entraînement rotatif (140) et l'actionneur (124).

12. Instrument (102) selon la revendication 11, dans lequel l'actionneur (124) inclut un mécanisme anti-rotation (114) qui empêche la rotation de l'actionneur par rapport à l'instrument autour de l'axe longitudinal mais qui permet une translation suivant l'axe longitudinal.

13. Instrument (102) selon la revendication 1, dans lequel l'instrument comporte une surface d'impaction (112) par l'intermédiaire de laquelle une force d'impaction peut être appliquée à l'instrument et un arbre central (110) qui est en communication avec la surface d'impaction de manière à transférer la force d'impaction au composant de cupule.

14. Instrument (102) selon l'une quelconque des revendications 1 à 14, dans lequel les éléments de mâchoire de la pluralité d'éléments de mâchoire (152, 154, 156, 158) sont des parties d'un élément de support de cupule (108) réalisé à partir d'une matière plastique.

15. Ensemble (100) destiné à être utilisé au niveau d'une procédure chirurgicale pour remplacer une articulation orthopédique, lequel comprend :
l'instrument (102) comme revendiqué selon l'une quelconque des revendications 1 à 14, et
un composant de cupule (104) d'une prothèse articulaire orthopédique, dans lequel chacun des éléments s'étendant transversalement sur les éléments de mâchoire de l'instrument est reçu dans un évidement respectif (186, 188, 190, 192) ménagé dans la paroi latérale orientée vers l'extérieur (184) du composant de cupule, et dans lequel les surfaces inférieures planes engagent la surface supérieure (182) du bord de la paroi du composant de cupule.
